(19) Europäisches Patentamt
European Patent Office
Office européen des brevets

(11) **EP 0 550 875 B2**

(12) **NEUE EUROPÄISCHE PATENTSCHRIFT**

(45) Veröffentlichungstag und Bekanntmachung des Hinweises auf die Entscheidung über den Einspruch:
**02.05.2007 Patentblatt 2007/18**

(51) Int Cl.:
*A61L 27/00* (2006.01)   *A61L 29/00* (2006.01)
*A61L 31/00* (2006.01)

(45) Hinweis auf die Patenterteilung:
**29.04.1998 Patentblatt 1998/18**

(21) Anmeldenummer: **92121785.7**

(22) Anmeldetag: **22.12.1992**

(54) **VERFAHREN ZUR HERSTELLUNG EINER PHARMAZEUTISCHE WIRKSTOFFE ENTHALTENDE IMPLANTIERBAREN VORRICHTUNG AUS EINEM POLYMEREN MATERIAL**

PROCESS FOR THE PRODUCTION OF AN IMPLANTABLE POLYMERIC DEVICE CONTAINING PHARMACEUTICAL AGENTS

PROCEDE DE PRODUCTION D'UN DISPOSITIF IMPLANTABLE EN MATERIAU POLYMERISABLE CONTENANT DES AGENTS PHARMACEUTIQUES

(84) Benannte Vertragsstaaten:
**BE CH DE ES FR GB IT LI NL**

(30) Priorität: **31.12.1991 DE 4143239**

(43) Veröffentlichungstag der Anmeldung:
**14.07.1993 Patentblatt 1993/28**

(73) Patentinhaber: **Schierholz, Jörg, Dr.Dr.**
**51427 Bergisch Gladbach (DE)**

(72) Erfinder: **Schierholz, Jörg, Dr.Dr.**
**51427 Bergisch Gladbach (DE)**

(74) Vertreter: **Meyers, Hans-Wilhelm et al**
**Patentanwälte**
**von Kreisler-Selting-Werner**
**Postfach 10 22 41**
**50462 Köln (DE)**

(56) Entgegenhaltungen:
**WO-A-87/03495          WO-A-89/04682**

- **J. of Hospital Infection 30, 201-210 (1995)**
- **Biomaterials 15 (12), 995-1000 (1994)**
- **Zentralblatt f. Bakteriologie 1996 (in press)**
- **Acta Neurochir (Wien) 133, 147-152 (1995)**
- **Clinical Materials 1996 (in press)**
- **Biomaterials 18 (8), 635-641 (1997)**
- **Hans-Georg Elias: Makromoleküle. 1975, 3. Auflage, Hüthing & Wepf Verlag, Basel, S. 173-185**
- **Gnamm / Fuchs: "Lösungsmittel und Weichmachungsmittel", Band I, "Physikalische Grundlagen und Eigenschaften der Lösungen von nieder- und hochmolekularen Verbindungen", 8. Auflage, 1980, Wissenschaftliche Verlagsgesellschaft mbH Stuttgart, Seiten 2, 3, 84, 85, 98-101, 324-327, 332, 333, 692, 693**
- **Biomaterials, 1998, Bd. 19, S. 2065-2074**

Bemerkungen:
Die Akte enthält technische Angaben, die nach dem Eingang der Anmeldung eingereicht wurden und die nicht in dieser Patentschrift enthalten sind.

EP 0 550 875 B2

## Beschreibung

Gegenstand der vorliegenden Erfindung ist ein Verfahren zur Herstellung einer vorgefertigten implantierbaren Vorrichtung gemäß Oberbegriff des Anspruchs 1.

Orthopädischer implantate werden oftmals nach Entnahme von Knochenteilen bei Carcinoma-Patienten oder Patienten mit rheumatischen Erkrankungen erforderlich. Es wäre daher von Vorteil, wenn solche Implantate mit Wirkstoffen beispielsweise mit Cytostatika oder Antirheumamittel wie Steroiden beladen werden könnten, um direkt am Implantatsort eine entsprechende Wirkung zu entfalten. Hohe systemische Dosierungen der Wirkstoffe zur Erzielung lokaler hoher Dosen könnten somit vermieden werden.

Auch im Rahmen einer Implantation eines By-Passes aus Fremdkörpermaterial ist es notwendig, die Komplikationsrate von 15 bis 25%. die durch thrombotische Verschlüsse hervorgerufen werden, zu senken. Hierbei kommen in erster Linie Wirkstoffe in Betracht, die die Thrombozytenaggregation vermindern, wie beispielsweise Acetylsalicylsäure. Diese Medikamente müssen auch für längere Zeiträume verabreicht werden. Bei den By-Pass-Patienten ist es zusätzlich indiziert, Vasodilatatoren zusätzlich freizusetzen, mit denen die Sauerstoffbilanz des geschädigten Herzens verbessert wird. Dabei kommen insbesondere Nitrate, wie Glycerolnitrat, Isosorbidnitrate in der Mono- oder Diform in Frage.

Ein nach wie vor großes Problem in der klinischen Medizin ist die Prävention von Infektionen bei Patienten, die Dauerimplantate oder für längere Zeit Implantate wie Katheter tragen müssen. So sind koagulase-negative Staphytokokken seit einigen Jahren zunehmend Hauptverursacher von Fremdkörperinfektionen (sogenannten Plastikinfektionen). Diese, lange Zeit als nicht-pathogen angesehenen Bakterien, spielen zum Teil die Hauptrolle bei der Infektion von Schrittmacherelektroden, Gelenkprothesen und allen möglichen Sorten von Kathetern. Zur Therapie dieser Fremdkörperinfektionen ist die Entfernung des Implantats notwendig, da eine systemische Chemotherapie mit Antibiotika alleine nicht wirksam ist, da unphysiologisch hohe Dosen des zur Vernichtung der Bakterien geeigneten Antibiotikums verwendet werden mußten.

Der erste Schritt der Besiedlung der Fremdkörperoberfläche nach Implantation ist die Adhäsion des Keimes, bevorzugt an Oberflächendefekten. Nach einigen Stunden der Adhäsion kann es mit der Proliferation der Bakterien zu der Produktion von extrazellulärem Schleim kommen, welcher ein starkes Penetrationshinderniss für das systemisch applizierte Antibiotikum darstellt. Nach verstärkter Vermehrung der Keime an der polymeren Oberfläche, kann es im Laufe der Zeit zu einer septischen Bakteriämie kommen. Um die Vermehrung der Keime an der Oberfläche zu unterbinden und damit kausal die Fremdkörperinfektion zu vermeiden, muß zur Zeit der Bakterienadhäsion ein stark wirksames Antibiotikum bereitgestellt werden. Dies kann durch die Inkorporierung eines geeigneten Chemotherapeutikums in die Polymermatrix gelingen, vorausgesetzt, daß das Chemotherapeutikum auch aus der Kunststoffmatrix herausdiffundieren kann. In diesem Fall kann dann die Freisetzung des Antibiotikums auf einen größeren Zeitraum ausgedehnt werden, damit für einen entsprechend längeren Zeitraum die Bakterienadhäsion bzw. Proliferation auf dem Polymer verhindert wird.

Die DE-A-34 29 038 betrifft ein resorbierbares Wirkstoffdepot auf Basis von Kollagen, dadurch gekennzeichnet, daß ein rekonstituiertes Kollagen mit Fließ- oder Schwammstruktur mit einem Aminoglykosid-Antibiotikum und einem bioresorbierbaren Polymeren mit polyanionischem Charakter beladen ist.

Auch die DE-A-33 34 595 betrifft ein resorbierbares Wirkstoffdepot auf Basis von Kollagen, welches dadurch gekennzeichnet ist, daß ein Kollagen, in dem weitgehend Depot und Struktur des natürlichen Materials enthalten ist, mit einem Aminoglykosid-Antibiotikum und einem bioresorbierbaren polymeren polyanionischem Charakter beladen ist.

Die DE-A-31 26 273.2 betrifft ein Verfahren zur Herstellung einer Tricalciumphosphat-Knochenkeramik und einem hiernach hergestellten Tricalciumphosphat-Keramikformkörper mit einer Mikro- oder Makroporosität für eine erhöhte Aufnahme eines Antiseptikums, Chemotherapeutikums oder Breitbandmikrobiozids und mit einer Außenbeschichtung für eine Langzeitfreigabe des Wirkstoffs, wo die Mikro- oder Makroporosität durch vor dem Brennvorgang der Keramik zugegebene anorganische und/oder organische mikro- oder makroporenbildende Substanz wie Zucker, Ammoniumbicarbonat und/oder Ammoniumcarbaminat, Calciumhydrogenphospat und dergleichen erhalten wird. Nach dem Brennvorgang wird der geformte Keramikformkörper mit einem Antiseptikum oder Chemotherapeutikum oder Breitbandmikrobiozid imprägniert und anschließend zur Erzielung einer verzögerten Freigabe des Antiseptikums oder des Chemotherapeutikums oder eines Breitbandmikrobizids zur einer vorgegebenen Freisetzungszeit mit einem Polymer in unterschiedlichen, genau auf die vorgegebene Freisetzungszeit abgestimmten Schichtdicke überzogen.

Die DE-A-20 22 117 betrifft ein Verfahren zur Herstellung von Knochenkitt aus Acrylharz, insbesondere zum festen Verbinden von Fremdkörperimplantaten wie künstlichen Gelenken, mit denen Fremdkörperimplantaten zugeordneten und diese haltenden Knochen, bei dem ein pulverförmiges polymeres Acrylharz, wie Polymethylmethacrylat, mit einem flüssigen monomeren Acrylharz, wie Methylmethacrylat, zu einer knetbar verarbeitbaren Substanz vermischt wird, die unter Polymerisation des Monomers aushärtet und dadurch gekennzeichnet ist, daß das pulverförmige Monomer vor dem Vermischen mit dem flüssigen Monomer mit einem pulverförmigen Antibiotikum, wie Gentamycin, vermischt wird.

Die DE-A-39 30 039 betriff eine infektvorbeugende Katheteranordnung mit einem Katheter, der ein starres oder flexibles Kanülenrohr mit einem am rückwärtigen Ende vorgesehen Anschlußstück aufweist, daß dadurch gekennzeichnet ist, daß durch eine an das Anschlußstück ansetzbare Einfüll- und Ansaugvorrichtung mit einem oder mehreren Wirkstoffreservoiren mit einem Gesamtvolumen, das dem Katheterfüllvolumen gegebenenfalls zuzüglich des Volumens von Zwischenstücken, insbesondere eines Dreiwegehahnes, entspricht, wobei dieses Volumen vollständig ausgefüllt ist und mindestens eines der Wirkstoffreservoire mit einer Substanz, enthaltend mindestens ein Antibiotikum bzw. Chemotherapeutikum bzw. antivirales Mittel, vorzugsweise Amminoglykosid, insbesondere Gentamycin, in mindestens minimalen Wirkkonzentrationen, gefüllt ist

Die WO 90/15586 beschreibt bioerodierbare Polymere für eine Arzneimittelabgabe in Knochen. Die bioerodierbaren Polymeren werden vollständig in nicht toxische Abbauprodukte zersetzt und zwar über eine klinisch sinnvolle Zeitdauer. Die bioerodierbaren Polymere, insbesondere Polyanhydride, Polyorthoester, Polyglycolsäuren, Polyessigsäuren und Copolymere derselben werden zur Abgabe der bioaktiven Stoffe wie Antibiotika, Chemotherapeutika, Inhibitoren von Angiogenesen und Knochenwachstumsfaktoren zur Abgabe direkt in den Knochen verwendet.

Auch durch Koppelung ionischer Antibiotika an mit ionischen Detergenzien wie Benzalkoniumchlorid oder Tridodecylammoniumchlorid versehenem Polymeroberflächen läßt sich eine antimikrobielle Wirkung erzielen. Weitere Möglichkeiten ergeben sich mit Beschichtung mittels Silberverbindungen, Silberantibiotikakomplexen oder Jodverbindungen. Dabei wird jedoch das geeignete Antibiotikum in der Polymermatrix durch Mischen von Chemotherapeutikum und Kunststoff während der Fabrikation des entsprechenden Materials realisiert.

Die WO/89/04682, beschreibt allgemein, daß Wirkstoffe durch vorherige Quellung eines Polymeren in die entsprechende Polymermatrix eingebracht werden können. Exemplifiziert wird das Verfahren an einem halbsynthetischen, antibiotischen Derivat des Rifampicin B (Rifampin) und der Droge Clindamycinhydrochlorid. Auf Seite 8 wird dann offenbart, daß die Konzentration im Bereich von 0,1% oder mehr liegt. Widersprüchlich ist, daß zum einen auf Seite 3 beschrieben ist, daß das "Swelling Agent" (Quellmittel) vollständig gelöst, einen oder mehrere antimikrobielle Agenzien enthalten soll, wohingegen die Seite 5 offenbart, daß die Wirkstoffe auch als kolloidale Suspensionen oder Emulsionen vorliegen könnten. Mittels Suspensionen oder Emulsionen lassen sich nur oberflächliche Beschichtungen der betreffenden Polymere einstellen.

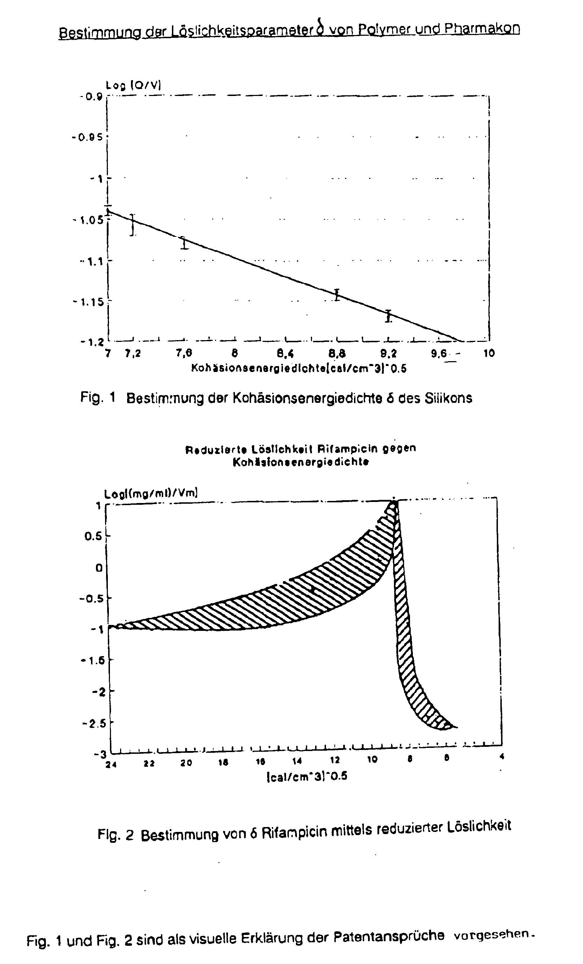

Die WO 87/03495 betrifft medizinische Vorrichtungen, die mit einer Kombination von antimikrobiell wirksamen Substanzen imprägniert sind durch die Einwirkung eines Quellmittels. Es wird weiter beschrieben, daß die aus Siliconkautschuk bestehenden medizinischen Vorrichtungen mit Rimactane (Rifampin USP) und Cleocinhydrochlorid (Clindamycinhydrochlorid) in Chloroform behandelt werden. Aus der Legene zur Figur 5 auf Seite 16 geht hervor, daß die Inkorporationsmenge ungefähr 0,1% jeweils gewesen ist Dies bedeutet, daß die Beladungseffizienz sehr ähnlich derjenigen ist, die in WO 89/04682 beschrieben worden ist

Nachteilig ist es in vielen Fällen, daß beim Produktionsvorgang der Polymere hohe Drucke und Temperaturen anfallen. Durch diese Parameter würde die Struktur des vorher erstellten Wirkstoffdepots und damit die Freisetzungskinetik des Wirkstoffs stark verändert werden. Erschwerend kommt zudem die Tatsache hinzu, daß die meisten pharmazeutischen Wirkstoffe nur wenig stabil gegen solche Einflüsse sind und der Wirkstoff schon beim Produktionsvorgang inaktiviert werden würde.

Das technische Problem der vorliegenden Erfindung besteht demnach darin, ein Verfahren zur Herstellung klinischer Gerätschaften bereitzustellen, die nachträglich mit pharmazeutischen Wirkstoffen imprägniert worden sind.

Das erfindungsgemäße Verfahren ist ein Verfahren zur Herstellung einer vorgefertigten implantierbaren Vorrichtung aus einem polymeren Material. Die Vorrichtung enthält pharmazeutische Wirkstoffe, wobei die vorgefertigte implantierbare Vorrichtung für eine bestimmte Zeitdauer mit einem organischen Lösungsmittel behandelt wird, wobei entweder der pharmazeutische Wirkstoff gleichzeitig anwesend ist oder nach einer bestimmten Zeitdauer in in einem ähnlichen oder gleichen organischen Lösungsmittel gelöster pharmazeutischer Wirkstoff mit dem vorbehandelten Implantat behandelt wird und daraufhin das oder die organischen Lösungsmittel abgedampft und die behandelten Implantate danach gegebenenfalls sterilisiert werden. In Weiterbildung des Standes der Technik wird erfindungsgemäß ein organisches Lösungsmittel und ein polymeres Material eingesetzt, das einen Flory-Huggins-Koeffizienten (Polymer Interaction Factor) 0,30 < x < 0,605 und/oder eine ähnliche Kohäsionsenergiedichte δ, die das polymere Material aufweist. Der zu inkorporiende pharmazeutische Wirkstoff weist eine ähnliche Kohäsionsenergiedichte im Verhältnis zum Polymer und Solvens auf. Durch die Abdampfbedingungen des organischen Lösungsmittels wird das Freisetzungsprofil des inkorporierten Wirkstoffs in wäßriger Lösung bestimmt, in dem bei schneller Entfernung des Lösungsmittels eine langsame anfängliche Freisetzung des Wirkstoffs aus der polymeren Matrix erreicht wird oder bei langsamer Entfernung des Lösungsmittels eine schnelle anfängliche Freisetzung des Wirkstoffs aus der polymeren Matrix erreicht wird.

Dieses technische Problem wird gelöst durch ein Verfahren mit den Merkmalen des Anspruchs 1. Unteranspruch 2 betrifft bevorzugte Ausführungsformen des erfindungsgemäßen Verfahrens.

Durch das organische Lösungsmittel wird das polymere Material gequollen. Vorzugsweise weist das organische Lösungsmittel (Quellmittel) einen X-Faktor < 0,5 (Flory Huggins Polymer-Interaction-Factor) und eine ähnliche Kohäsionsenergiedichte wie das Polymer auf. Es ist vorteilhaft, als Quellmittel eine möglichst nicht toxische, chemische Substanz zu verwenden, die eine möglichst geringe Residualmenge an Solvens nach dessen Entfernung in der Polymermatrix zurückläßt. Quellmittel mit einem Faktor wesentlich unter 0,3 ergeben eher hohe Residualmengen an Solvens. Es ist aufgrund dieser Verhältnisse vorteilhaft, Quellmittel mit niedrigem Siedepunkt zu wählen.

Der zu inkorporierende pharmazeutische Wirkstoff sollte zumindest zwei Eigenschaften besitzen:

- eine hinreichende Wirksamkeit für die betreffende Indikation, zum Beispiel bei Antibiotika

- eine ausreichende antimikrobielle Wirksamkeit gegen die infektionsverursachende Spezies, eventuell mit breitem Wirkungsspektrum.

- ausreichende physio-chemische Eigenschaften, wie ähnliche Kohäsionsenergiedichte im Verhältnis zum Polymer und Quellmittel und ein nicht zu großes Molekulargewicht zur Gewährleistung eines ausreichend großen Diffusionkoeffizienten im gequollenen und auch ungequollenen Polymer.

Als Polymeren kommen die in der Klinik üblicherweise verwendeten Kunststoffe in Betracht. Dies sind insbesondere Polyethylen, Polypropylen, vernetzte Polysiloxane, Polyurethane, Polymere auf Acrylatbasis, Cellulose und Cellulosederivate (Nähte), Polycarbonate, Tetrafluorethylenpolymere und Polyethylenterephthalate. Die implantierbaren Vorrichtungen sind insbesondere Katheter jedweder Art, intraokulare Linsen. Kontaktlinsen und ähnliche aus Kunststoffen herstellbare Implantate. Als pharmazeutischen Wirkstoffe kommen insbesondere solche Antibiotika wie Rifampicin- oder Gentamycin-Derivate, Cytostatika, Antirheumamittel wie Steroide, infrage.

Für die Controlled-Release-Systeme der vorliegenden Erfindung für die lokale Freisetzung von Chemotherapeutika mit den üblichen Vorteilen im Vergleich zur systemischen Applikation eignen sich auch Cytostatika, da insbesondere die Nebenwirkungen dieser Wirkstoffe auf alle schnell proliferierenden Gewebe sehr stark sind. Solch ein System eignet sich daher insbesondere für die Versorgung von Patienten mit Knochenkerzinomen mit mäßig weit fortgeschrittener Metastasierung. Als solche Substanzen kommen in Betracht Cyclophosphamid, Cisplatin, Melphalon, Methothrexat, Adriamycin, Bleomycin, Vinblastin sowie Vinchristin, Hormone und Antihormone, beispielsweise bei einer Mammaplastik in der Postmenopause mit den Antiestrogenen Tamoxifen und je nach Rezeptorendifferenzierung bei fortgeschrittener Metastasierung mit Gestagenen und/oder Estrogenen. Beim fortgeschrittenen nicht operablen Prostatakarzinom mit dem Antiandrogen Cyproteronacetat oder einem Antagonisten des LH-Releasing-Hormons, wie Buselerin, Ketoconazol. Zudem können bei Polyarthritis und schwerem systemischen Lupus erythomatodes Azathioprin und Methotrexat bei schwerer psoriatischer Arthritis lokal Verwendung finden. Auch hierbei können die systemischen Schäden wie Nephrosen und Hepatosen, Panzytopenien, Haarausfall und Nausea vermindert werden.

TABELLE 1

| Substanz | Quellmittel | Polymer |
|---|---|---|
| Cyclophosphamid | Ethanol, Chloroform, Toluol, Aceton, Dioxan | PUR, PDMS, Poly-Polycarbonat, Polyethylen |
| Tamoxifen | Chloroform, Hexan | PDMS, PMMA, Polyethylen |
| Buselerin | Methanol, Ethanol | PUR, Polycarbonat, Cellulose |
| Ketoconazol | Chloroform, Methanol | PDMS, PMMA, Polycarbonat, Polyethylen |

Beim Einsatz orthopädischer Implantate auf Polymerbasis im Rahmen immunologischer Erkrankungen wird das Grundleiden bzw. das Symptom nicht beseitigt. Eine lokale Freisetzung von Antiphlogistika und/oder sogenannter Basistherapeutika schafft hier Abhilfe. Die In Frage kommenden Therapeutika sind Glukokortikoide, Prednison, Prednisolon, Methylprednisolon, Triamcinolon, Dexamethanson, Betamethason und Fluocortolon sowie nichtsteroidale Antiphlogistika, wie die Arylessigsäurederivate (Indometacin), Arylpropionsäurederivate (Ibuprofen), Oxicame (Piraxicam), Pyrazolderivate (Phenylbutazon), Anthranilsäurederivate (Flutenaminsäure) und Acetylsalicylsäure. Es empfiehlt sich, zusätzlich Antibiotika mit freizusetzen, um Fremdkörperinfektionen vorzubeugen, da lokal das Immunsystem supprimiert wird. Zusätzlich können bei der Anwendung besonders der Glukokortikoide zur lokalen Osteoporoseverminderung und Knochenstabilisierung anabole Steroide und F-Salze mit freigesetzt werden. Als Substanz/Quellmittel und Polymersystem kommen die in der folgenden Aufstellung genannten in Frage.

TABELLE 2

| Substanz | Quellmittel | Polymer |
|---|---|---|
| Diclofenac | DMF, Ethanol | PUR, Polycarbonat |

(fortgesetzt)

| Substanz | Quellmittel | Polymer |
|---|---|---|
| Indometacin | Chloroform | PMDS, PMMA* |
| Ibuprofen | Chloroform, Ethanol, Aceton, Ether | PDMS, PMMA*, Teflon* |
| Piroxicam | Methanol | PUR, Polycarbonat, Cellulose* |
| Phenylbutazon | Chloroform, Aceton | PDMS, PMMA* |
| Acetylsalicylsäure | Ethanol | PMMA*, Polyester |
| Glukokortikoide (Steroide) | Ethanol, Methanol, Aceton, Chloroform mit Ausnahme von Triamcinolonacetonid, welches gut in DMF löslich ist | Zur Quellung können fast alle genannten Polymere im Bereich mittlerer Kohäsionsenergiedichten verwendet werden, da es sich bei den Steroiden um amphiphile Stoffe handelt |
| * nicht erfindungsgemäß | | |

Bei den sogenannten Basistherapeutika handelt es sich vorwiegend um Goldpräparate wie Goldthiopolypeptid, Goldthioglukose, Natriumgoldthionalat und Auranofin. Als Controlled-Release-System gemäß der Erfindung eignet sich als Quellmittel für Auranofin insbesondere Chloroform, wenn Materialien aus PDMS, verwendet werden. Die Nebenwirkungen dieser Präparate, die bei systemischer Applikation sehr vielfältig sind und Symptome wie Dermatitis, Stomatitis, toxische Blutreaktionen, Nierenschädigungen und Hepatosen können durch die erfindungsgemäße Vorgehensweise vermieden werden.

Der Vorteil der Imprägnierung orthopädischer Implantate liegt auch hier in der Verminderung der Nebenwirkungen der ansonsten notwendigen systemischen Applikation der einzelnen Wirkstoffe. Die Nebenwirkungen umfassen Schwächung der Immunabwehr (Phagozytosehemmung, Opportunistische Infektionen), Aktivierung von Magenulzera, Osteoporose, Elektrolytstörungen, Myopathien, ZNS-Reaktionen und verzögerter Wundheilung.

Bei der Verwendung von wirkstoffimprägnierten By-Pass-Implantaten, die Thrombozytenaggregation hemmende Substanzen enthaltend, werden in vorteilhafterweise die Nebenwirkungen der entsprechenden Medikamente, wie Bronchospasmus, Magen-Darm-Blutungen, Aktivierung peptischer Ulzera, Nausea und Elektrolytstörungen bei systemischer Anwendung vermieden. Sind ebenso Vasodilatatoren in den Implantaten enthalten, wird die Sauerstoffbilanz des geschädigten Herzens verbessert. Als Wirkstoffe kommen hier insbesondere Nitrate in Frage.

Bei lokaler Anwendung der Betarezeptorenblocker in den Controlled-Release-Systemen der vorliegenden Erfindung werden Nebenwirkungen der Substanzen, wie Oxoprenolol, Propenolol, Timolol, Sotalol, Nadolol, Metoprolol, Atenolol, Bisoprolol, Betaxolol, Pindool, Cortiolol und anderen verhindert. Diese Anwendungen äußern sich in Kopfschmerzen, Blutdruckabfall und Synkopen.

TABELLE 3

| Substanz | Quellmittel | Polymer |
|---|---|---|
| Acetylsalicylsäure | Ethanol | PUR, PMMA*, Polyester |
| Isosorbidnitrat | Chloroform, Aceton | PDMS, Teflon*, PMMA* |
| Nifedipin | Chloroform, Aceton | PDMS, Teflon*, PMMA* |
| Verapamil | Chloroform, Aceton, Ethylacetat | PDMS, Teflon*, PMMA*, Polyethylen* |
| Nadolol | Methanol | PUR, Polycarbonat*, Cellulose*, Polyethylentherephtalat |
| Metropolol | $H_2O$, Ethanol | Cellulose*, PUR |
| Pindolol | höhere Alkohole | PUR, Polycarbonat, Cellulose* |
| * nicht erfindungsgemäß | | |

Die den pharmazeutischen Wirkstoff enthaltende implantierbare Vorrichtung aus einem polymeren Material ist erhältlich durch Behandlung der entsprechenden vorgefertigten implantierbaren Vorrichtungen aus polymerem Material, denen kein Wirkstoff zugesetzt ist. Dabei wird mit einem organischen Lösungsmittel die implantierbare Vorrichtung aus einem polymeren Material-gequollen, wobei die Polymermatrix so, verändert wird, daß pharmazeutische wirksame Substanzen mit, relativ zum Polymermaterial der Implantate, geringer Molekülmassen in das polymere Material des Implantats eindringen können. Nach dem Entfernen des organischen Lösungsmittels wird der Wirkstoff im polymeren Material der implantierbaren Vorrichtung eingeschlossen. Nach Kontakt mit physiologischem Medium, wie beispielsweise nach der Implantierung, wird die dann in der implantierbaren Vorrichtung enthaltende pharmazeutisch wirksame Substanz durch Diffusion wieder freigesetzt.

Für implantierbare Vorrichtungen aus vernetztern Polydimethylsiloxan, das mit hydrophobierter Kieselsäure als Füllstoff verstärkt sein kann, hat sich als organisches Lösungsmittel (Quellmittel), insbesondere Chloroform bewährt. Bei dieser Polymeren-/Lösungsmittelkombination hat sich Rifampicin, welches üblicherweise als Tuberkulostatikum Verwendung findet, sich aber durch eine äußerst hohe Wirksamkeit gegenüber Staphylokokken auszeichnet, als besonders geeignet erwiesen.

Die Inkorporierungsmenge an pharmazeutischem Wirkstoff kann in Abhängigkeit von Konzentration und Quelldauer variiert werden. Durch Temperatursteigerung bis zum Siedepunkt des verwendeten organischen Lösungsmittels können Inkorporierungsmengen bis zu 10 Massen-% erzielt werden. Allgemein kann auch durch Variation der Abdampfbedingungen des organischen Lösungsmittels, wie durch Temperatur- und Druckbeeinflussung das Freisetzungsprofil des inkorporierten Wirkstoffs in wäßriger Lösung signifikant und reproduzierbar verändert werden, auch die Konzentration des Quellmittels und Quelldauer der Wirkstofffreisetzung.

Bei dem oben beispielhaft angeführten nicht erfindungsgemäßen Polysiloxan/Chloroform/Rifampicin-System wurde durch Erhitzen bis zum Siedepunkt des Quellmittels eine Inkorporierungsmenge von 9 Massen-% Rifampicin im polymeren Material erzielt. Mittels der Abdampfbedingungen läßt sich das Freisetzungsprofil des Antibiotikums oder der Antibiotikakombination steuern.

Wird das Quellmittel relativ schnell entfernt, etwa durch Abdampfen bei Unterdruck und jeweiliger Siedetemperatur, erreicht man eine relativ langsame anfängliche Freisetzung des Wirkstoffs, wohingegen eine langsame Entfernung des Quellmittels zu einer schnellen anfänglichen Freisetzung des Wirkstoffs aus der polymeren Matrix erreicht wird.

Zur Bestimmung der beispielsweise antimikrobiellen Aktivität der behandelten implantierbaren Vorrichtungen aus polymerem Material wurde in vitro die Messung der Adhäsion von Staphylokokken in Abhängigkeit von der Zeit gemessen. Bei einer Inkorporierungsrate von nur einem Massen-% Antibiotikum ließ sich über einen Zeitraum von etwa drei Tagen die Keimzahl der Staphylokokken unter die Meßgrenze reduzieren. Bei der maximalen Inkorporierungsrate von 9 Massen-% Rifampicin im polymeren Material wurde die Keimreduktion unter die Bestimmungsgrenze bereits nach 5 Stunden Inkubationszeit erreicht

Hinsichtlich der freigesetzten Antibiotikamenge sind die bis zur Sättigung beladenen implantierbaren Vorrichtungen aus polymerem Material mindestens über einen Zeitraum von drei Wochen nach Implantation stark antimikrobiell wirksam und somit auch als länger implantierbare antiinfektiöse Fremdkörper für die medizinische Anwendung geeignet.

Nach längerer Dauer der Implantierung werden biokompatible Polymere nach und nach vollständig fibrosiert und je nach Gewebeart mit Endothelzellen besiedelt. An solch einem System ist eine Bakterienadhäsion lange nach Implantierung sehr unwahrscheinlich, d. h. die vulnerable Phase des Polymers liegt höchstens bei einigen Wochen.

Das erfindungsgemäße Verfahren zur Herstellung der implantierbaren Vorrichtungen, die mit pharmazeutischen Wirkstoffen imprägniert sind, weist als erste Verfahrensstufe die Behandlung des bereits fertiggestellten implantierbaren klinischen Gerätes mit einem organischen Lösungsmittel (Quellmittel) auf. Die Vorrichtung wird für eine bestimmte Zeit mit diesem Quellmittel, wahlweise bei erhöhter Temperatur, behandelt. Dabei kann gleichzeitig bereits ein in dem entsprechenden Lösungsmittel lösliches oder durch Zusatz von üblichen Lösungsvermittlern löslich löslich gemachter Wirkstoff zugegen sein. Es ist jedoch auch möglich, vorgequollenes polymeres Material in eine Lösung des zu verwendenden Wirkstoffs zu geben, um dann wie oben beschrieben zu verfahren.

Es ist besonders vorteilhaft bei endothermen Quellsystemen die Quellung und Imprägnierung mit Antibiotikum des polymeren Materials bei Siedetemperatur des entsprechenden Quellmittels durchzuführen. Vorzugsweise wird die zu behandelnde implantierbare Vorrichtung unter Rückfluß mit dem Lösungsmittel, gegebenenfalls in Gegenwart des Antibiotikums, erhitzt. Danach wird das Lösungsmittel gegebenenfalls unter reduziertem Druck abgedampft.

Es hat sich als vorteilhaft erwiesen, daß das organische Lösungsmittel (Quellmittel) und das polymere Material einen Flory-Huggins-Polymer-Interaction-Koeffizienten X < 0,5 aufweist. Das Mischen eines Lösungsmittels 1 und eines Polymers 2 kann als quasichemische Reaktion aufgefaßt werden. Dabei wird ein Paar von Lösungsmittelkontakten 1-1 mit dem Kontaktpaar 2-2 von Polymerbausteinen zu zwei Paaren 1-2 umgesetzt. Die Änderung der Wechselwirkungsenergie beträgt entsprechend $\delta\varepsilon = e_{1-2} - 0{,}5(\varepsilon_{1-1} + \varepsilon_{2-2})$. Die Mischungsenthalpie ist durch das Produkt aus $\delta e$ und der Zahl $N_{1-2}$ der Paare 1-2 gegeben, die wiederum von der Zahl $N_G$ der Gitterplätze, der Zahl Z der nächsten Nachbarn um das 1-1 Paar und der Wahrscheinlichkeit abhängt, daß der nächste Gitterbauplatz von einem Lösungsmittel oder einem Polymerbaustein besetzt ist.

Diese Warscheintichkeiten sind durch die Volumenbrüche $\phi_1$ und $\phi_2$ gegeben, so daß $\delta H_{mix} = N_G {}^* Z {}^* \phi_1 {}^* O_2 {}^* \delta\varepsilon$.

Hierauf wird der Wechselwirkungsparameter definiert, welcher Z und 5 $\varepsilon$ erfaßt und sich auf die thermische Energie bezieht:

6

$$X = Z * \delta_{\epsilon} / K_B * T$$

Hierbei wird angenommen, daß die Reaktion 1-1 und 2-2 ohne Volumenänderung vorläuft und rein enthalpischer Natur ist. Allerdings verlaufen die beiden Reaktionen mit Schwingungs- und Rotationsanteilen, woraus folgt, daß der Flory-Huggins-Koeffizient noch einen entropischen Anteil hat. Es ergibt sich in der Praxis, daß die Gibbs-Duhem-Mischungsenthalpie bei X = 0,5 immer negativ ist; eine Entmischung ist kaum möglich. Zusätzlich stellt der Wert 0,605 einen kritischen Wechselwirkungsparameter dar, oberhalb dieses Wertes wird das chemische Potential positiv und es wird thermodynamisch eine Phasentrennung erwartet. Somit ist für ein gutes Löse- oder Quellmittel die Voraussetzung ein X-Wert von 0,5.

Tabelle einiger Lösungsmittel für PDMS und die entsprechenden Flory-Huggins-Parameter

| | | Polymerwechselwirkungsfaldor n (geringe - hohe Solvenskonzentration) |
|---|---|---|
| Lösemittel: | Cyclohexan | 0,41 - 0,46 |
| | Cyclopentan | 0,42-0,46 |
| | Diisobutylketon | 0,49 |
| | Hexadimethyldisiloxan | 0,25 - 0,3 |
| | Octamethyltrisiloxan | 0,17 - 0,22 |
| | n-Octan | 0,51 - 0,42 |
| | n-Pentan | 0,4-0,43 |
| | Toluol | 0,72 - 0,45 |

Sehr oft steigt X mit steigender Polymerkonzentration bei schwachen Lösungsmitteln. In vielen Fällen bei guten Lösungsmitteln ist X unabhängig von der Konzentration: In sehr wenigen Fällen bei exothermen Systemen fällt X mit steigender Polymerkonzentration. Bei hohen Polymerkonzentrationen sind die Abhängigkeiten von X zum Volumenbruch nicht mehr linear, im Falle von zum Beispiel Polyvinylmethoxyacetal wird ein Maximum bei hoher Polymerkonzentration durchlaufen. Bei apolaren, flexiblen Polymeren wie PDMS wird ein lineares Verhalten erwartet.

Eine Abnahme von X mit wachsendem Polymervolumenbruch deutet auf eine zunehmende Abstoßung zwischen den Polymerbausteinen hin bzw. auf Änderungen der Struktur geordneter Lösungsmittel, zum Beispiel durch hydrophobe Effekte bei Polyvinylmethoxyacetal oder durch Ausbildung von lyotropen Flüssigkeitskristallen bei Zellulosenitrat.

Voraussetzung zur Inkorporierung eines Wirkstoffes in ein Polymer zum Zwecke der Freisetzung ist eine entsprechende Löslichkeit. Da man die Löslichkeit hier vom Wirkstoff in ein vernetztes Polymer nicht direkt bestimmen kann, werden die Löslichkeitsparameter $\delta_x$ über die monomeren Einheiten des Polymers abgeschätzt. Diese Löslichkeitsparameter werden über die Kohäsionsenergiedichten berechnet, wobei

$$\delta_1 2 = N_L * Z * \epsilon_{1\ 1} / 2V_1 \ (cal/cm^3)$$

$\delta^2$ = Kohäsionsenergiedichte
$V_1$ = Molvolumen.

Zieht man von der Verdampfungsenthalpie die gegen den Außendruck zu leistende Arbeit ab, so erhält man die Verdampfungsenergie pro Einheitsvolumen.

Polymere haben eine Verdampfungsenergie von $E_2 = V_2 * \delta_2 2$. In der Praxis liegen die $\delta$-Werte zwischen 7 (Aliphaten) und 23,4 (Wasser). Für polare Substanzen gelten außer dispersen Kräften auch Dipol-Dipol-Wechselwirkungen und H-Brückenbindungen. Somit gilt die Erweitung des Terms auf $\delta^2 = \delta^2_{dispers} + \delta^2_{polar} + \delta^2_{H\text{-}Brücke}$.

Bei Polymeren erhält man $\delta$ außer über die Abschätzung monomerer Einheiten über die Grenzviskositätszahlen oder Quellungsgrade linearer Makromoleküle. Da im speziellen Fall eines vernetzten Silikonkatheters kein lineares Polymer im strengen Sinn mehr vorliegt, entfallen diese beiden Methoden. Somit kann über die Löslichkeitsparameter die Löslichkeit eines Stoffes im vernetzten Polymer abgeschätzt werden.

Vorzugsweise geht man erfindungsgemäß so vor, daß die Löslichkeiten des Antibiotikums in 20 verschiedenen Lösungsmitteln bestimmt werden und über einen Vergleich tabellarisch aufgelisteter $\delta$-Werte die Kohäsionsenergiedichten für den dispersen, polaren und stark attraktiven Bereich (Wasserstoff-Brücken) des Antibiotikums ermittelt werden.

Tabelle der Hildebrand-Parameter apolarer Lösungsmittel in Relation zu Wasser und Methanol

| | $\delta_1$ | $\delta_d$ | $\delta_P$ | $\delta_H$ |
|---|---|---|---|---|
| Lösungsmittel Cyclohexan | 8,18 | 8,18 | | |
| Lewis-Säuren: | | | | |
| $CCl_4$ | 8,65 | 8,65 | | |
| $CHCl_3$ | 9,33 | 8,75 | | |
| $CH_2Cl_2$ | 9,73 | 8,72 | | |
| Lewis-Basen: | | | | |
| Diethylether | 7,61 | 7,05 | 1,4 | 2,5 |
| Trimethylamin | 7,4 | | | |
| Methanol: | 14,6 | 7,42 | | |
| Wasser: | 23,4 | 7,6 | 7,8 | 20,4 |

Zusätzliche Lösungsmittel für Siloxane, insbesondere PDMS sind Monochlor- und Dichlorbenzol, o-F-Toluol, Ethylbromid, 1,2-Dimethoxyethan, Methylethylketon unter Erhitzen, Ethylacetat, Isopropylacetat, Amylacetat, Cyclohexylacetat.

Theta-Lösungsmittel

Bei einer bestimmten Temperatur (Theta-Temperatur) kompensieren sich die Mischungsentropie und -enthalpie. Diese Lösung bezeichnet man als pseudoideale Lösung, das physikalische Verhalten als ideales Verhalten. Die Theta-Temperatur ähnelt somit der Boyle Temperatur idealer Gase. Interessant für die Quellung in PDMS sind somit auch die Thetamischungen:

| | |
|---|---|
| Aceton/Toluol | 6 : 5 (29°C) |
| Benzylalkohol/$CHCl_3$ | 12,8 : 87,2 (25°C) |
| Cyclohexanol/Toluol | 34 : 66 (25°C) |
| Ethanol/n-Heptan | 49 : 51 (63°C) |

Nichtlösermischungen ergeben manchmal gute Mischlöser und Mischungen von guten Lösern zusammen manchmal Nichtlöser. Aus diesen Beispielen ist erkennbar, daß es gewisse Abweichungen von der linearen Beziehung von Löslichkeit und der Löslichkeitsparameter gibt, jede Kombination muß für sich geprüft werden.

Vorzugsweise werden für Polyurethane je nach zu erhaltenen Quellungsgrad (Gel-Lösung) eher aprotische, polare Lösungsmittel in Betracht gezogen: DMF, DMA, Methylethylketon, HMPT, DMSO, Dioxan (heiß), m-Kresol, Trikresol, n-Propanol, Dichloressigsäure. Als bevorzugt haben sich die folgenden Mischungen herausgestellt: Aceton/DMF 21 : 79, Dichlorethan/Diethylenglykol 80 : 20, $CH_3OH/CCl_4$ 80 : 20, $CCl_4$ mit m-Kresol bzw. Cyclohexan bzw. Ethylacetat, DMF bzw DMA mit versch. Ether bzw. Aceton.

Lösungsmittel für Polycarbonate sind vorzugsweise n-Heptan, n-Octan, n-Hexan, THF, o-Dichlorbenzol, Benzol, $CHCl_3$, Aceton, Dioxan, DMF, m-Kresol, Cyclohexanon, Pyridin, Ethylacetat oder Mischen wie $CHCl_3$/Methanol, Methylendichlorid/n-Hexan bzw. n-Heptan.

Polyethylenterephthalate lösen sich in Cyclohexan/Dichloressigsäure.

Prinzipiell als Wirkstoff einsetzbar sind zum Beispiel alle Antibiotika, welche in ihrem Erregerspektrum Staphylokokken beinhalten und sich durch jeweils geeignete Löslichkeitsparameter auszeichnen. Für PDMS werden vorzugsweise Rifampicin, Fusidinsäure, Erythromycinbase, Clindamycinstearat, Mupirocin, Trimethoprim (bezogen auf Reinstsubstanzen ohne Zusätze) eingesetzt. Mit Hilfe eines Löslichkeitsvermittlers kann das Spektrum auf Gyrasehemmer, Gentamycinbase. Isoxazylpenicilline und entsprechende Cephalosporine (Cephomandolformiat) erweiter werden, obwohl die Quellung im einfachen Solvens bevorzugt ist.

Für PUR werden vorzugsweise eingesetzt Isoxyzylpenicilline, Gentamycinbase, Cephalosporine der 3. oder 4. Generation und Ciprofloxacin oder ein anderer Gyrasehemmer, welcher über entsprechende Löslichkeitsparameter verfügt.

Cephalosporine zum Einsatz.

Es ist erfindungsgemäß möglich, alle pharmazeutischen Wirkstoffe zu kombinieren, sofern sich die Wirkungen nicht antagonisieren und ähnliche Löslichkeitsparameter vorhanden sind. So wird bei einer Kombination von Antibiotika in jedem Fall wird eine eventuelle Resistenzverminderung eintreten, im positiven Falle zusätzlich ein Synergismus der Wirkungen. Zusätzlich wird zum Beispiel leicht ein erweitertes antimikrobielles Spektrum erreicht, die Wirkgeschwindigkeit ist oft vergrößert, und letztendlich bei Summation der oben genannten Gründe wird nicht soviel Wirksubstanz benötigt. Günstige Kombinationen von Antibiotika sind insbesondere Rifampicin-Gyrasehemmer (Ciprofloxacin, Pefloxacin), Rifampicin-Muptrocin, Rifampicin-Clindamycin, Rifampicin-Gentamicin. Auch andere Kombinationen sind möglich.

Das erfindungsgemäße Verfahren ermöglicht die kontrollierte Einschleusung von pharmazeutischen Wirkstoffen, zum Beispiel von antibiotisch wirksamen Substanzen, Cytostatika, Antirheumamittel, in bereits vorgefertigte klinische Gerätschaften. Dadurch wird es ermöglicht, an die Einsatzgebiete optimal angepaßte Wirkstoffe in die entsprechenden Gerätschaften zu inkorporieren. Je nach Anwendungsbereich ist es demnach zum Beispiel auch möglich, die gewünschte Menge an Antibiotikum zu regulieren. Üblicherweise ändern sich die mechanischen Eigenschaften der Gerätschaften aus polymerem Material in Abhängigkeit von der Wirkstoffmenge. So sinkt beispielsweise im System Chloroform/Siloxan/Rifampicin der Elastizitätsmodul, die Reißfestigkeit und das Dehnungsvermögen des Elastomers. Die mechanischen Eigenschaften, insbesondere im Katheterbereich, werden jedoch nicht so stark beeinträchtigt, daß der Einsatzbereich der Katheter in Frage gestellt wäre. Sollen die Gerätschaften vor ihrem Einsatz sterilisiert werden, müssen seibstverständlich Wirkstoffe eingesetzt werden, die die thermische Behandlung der Sterilisation umbeschadet überstehen. Kaltsterilisationsverfahren wie radioaktive Bestrahlung sind daher besonders bevorzugt. Auch hat sich das Plasmasterilisationsverfahren, zum Beispiel im Vakuum mit Argon 4 GHz, 600 Watt bewährt.

Vorteilhaft bei den, mit Antibiotika imprägnierten Kathetern, ist deren Langzeiteinsatz. Im Tiermodell wurde anhand von 10 Kaninchen, die mit der Schwellendosis des Keimes Staphylokokkus epidermidis RP 62 Im Ventrikelraum infiziert wurden und denen Antibiotika enthaltende Katheter, die erfindungsgemäß behandelt worden waren. Implantiert. Keines der Tiere zeigte Zeichen einer relevanten klinischen Entzündung: Liquor, Hirngewebe und Blut der Kaninchen blieben steril.

Die Erfindung wird anhand der folgenden Beispiele näher erläutert:

Beispiel 1

Zur Kontrolle der Sterilität der Operationsbedingungen bei der Implantierung eines 1 cm langen PDMS-Schlauches in den Ventrikelraum des Säugers wurden 10 Kaninchen ohne Anzeichen einer nachfolgenden Sepsis in einem standardisierten Verfahren operiert. Weder aus dem Katheter noch im Hirngewebe, Liquor oder Blut ließen sich Bakterien finden.

Zur Ermittlung der Schwellendosis bei der Implantatkontamination und sicherer Kolonienbildung auf dem Kunststoff wurden 26 Kaninchen mit steigenden Dosen von $10^2$ bis $10^8$ Keimen des normalerweise apathogenen Stammes Staphylokokkus epidermidis $RP_{62}$ bei der intrathekalen Implantierung des Katheters infiziert. Auf insgesamt 77% der Katheter konnte nach 1 - 2 Monaten Implantationsdauer der Keim isoliert werden, ab einer Kontaminationsdosis von $10^6$ Bakterien waren auf jedem Katheter Staphylokokkenkolonien zu finden.

Bei 15 Kaninchen wurden die 9% Rifampicin enthaltenden Katheterstücke mit jeweils der Schwellendosis in den Hirnventrikel implantiert. Keines der Tiere wies nach 1 bis 8 Wochen klinische Zeichen einer Sepsis auf, weder auf dem Silikon noch im Hirngewebe, Blut und Liquor ware Keime zu finden.

Beispiel 2 (nicht erfindungsgemäß)

Ein PDMS-Katheter wird bis zum Gleichgewicht in $CHCl_3$ bei 62°C (20 Min.) vorgequollen, dann erfolgt eine weitere Quellung in 42% Rifampicin-$CHCl_3$-Lösung bei 62°C (4 Stunden). Daraufhin wird das Solvens bei 60°C und 0,1 mbar abgezogen. Unter diesen Abdampfungbedingungen des Chloroforms erzielt man eine Reduktion des anfänglichen "Burst-Effects", die hohe Initialfreisetzung wird vermindert und der Wirkstoff wird in signifikanter Weise über einen längeren Zeitraum freigesetzt. Bei einer langsameren Kinetik der Evaporation (Temperatur bis 60°C. Druck 1.0 mbar) erhält man eine größere Oberflächenkonzentration des Antibiotikums, somit eine geringere Beladung der polymeren Matrix. Daraus resultiert ein stärkerer "Burst-Effect".

**Patentansprüche**

1.   Verfahren zur Herstellung einer vorgefertigten implantierbaren Vorrichtung aus einem polymeren Material, ausgewählt aus der Gruppe bestehend aus PDMS, Polyurethanen, Polycarbonaten, Polyethylenterephthalaten die pharmazeutische Wirkstoffe, ausgenommen antimikrobielle Wirkstoffe mit quartären Ammoniumgruppen, enthält, wobei die vorgefertigte implantierbare Vorrichtung für eine bestimmte Zeitdauer mit einem organischen Lösungsmittel behandelt wird, wobei entweder der pharmazeutische Wirkstoff gleichzeitig anwesend ist oder nach einer bestimmten Zeitdauer ein in einem ähnlichen oder gleichen organischen Lösungsmittel gelöster pharmazeutischer Wirkstoff mit dem vorbehandelten Implantat behandelt wird und daraufhin das oder die organischen Lösungsmittel abgedampft werden und die behandelten Implantate danach gegebenenfalls sterilisiert werden, **dadurch gekennzeichnet, dass** das organische Lösungsmittel und das polymere Material einen Flory-Huggins-Koeffizienten (Polymer-Interaction Factor) 0,30 < X < 0,605 und/oder eine ähnliche Kohäsionsenergiedichte wie das polymere Material aufweist,

- der zu inkorporierende pharmazeutische Wirkstoff eine ähnliche Kohäsionsenergiedichte im Verhältnis zum Polymer und Solvens aufweist,
- die Abdampfbedingungen des organischen Lösungsmittels das Freisetzungsprofil des inkorporierten Wirkstoffs in wässriger Lösung bestimmen, indem bei schneller Entfernung des Lösungsmittels eine langsame anfängliche Freisetzung des Wirkstoffs aus der polymeren Matrix erreicht wird, oder bei langsamer Entfernung des Lösungsmittels eine schnelle anfängliche Freisetzung des Wirkstoffs auf der polymeren Matrix erreicht wird, mit der weiteren Maßgabe, dass eine implantierbare Vorrichtung aus
- PDMS mit Chloroform und Fusidinsäure, Erythromycinbase, Clindamycinstearat, Mupirocin, Trimetoprim, behandelt wird,
- Polyurethan mit DMF, DMA, Methylethylketon, HMPT, DMSO, Dioxan (heiß), m-Kresol, Trikresol, n-Propanol, Dichloressigsäure oder Mischungen aus Aceton DMF, Dichlorethan/Diethylenglykol, Methanol/-Tetrachlorkohlenstoff, Tetrachlorkohlenstoff/mKresol, Tetrachlorkohlenstoff/Cyclohexan, Tetrachlorkohlenstoff/Ethylacetat, DMF/DMA mit verschiedenen Ethern oder Aceton zur Quellung gebracht wird,
- Polycarbonat mit n-Heptan, n-Octan, n-Hexan, THF, o-Dichlorbenzol, Benzol, Chloroform, Aceton, Dioxan, DMF, m-Kresol, Cyclohexanon, Pyridin, Ethylacetat oder Mischungen wie Chloroform/Methanol, Methylenchlorid/n-Hexan bzw. n-Heptan und
- Polyethylenterephthalat mit Cyclohexan/Dichloressigsäure zur Quellung gebracht wird.

2. Verfahren nach Anspruch 1, wobei unter Zuhilfenahme geeigneter Lösungsvermittler, wie Dimethylformamid, Ethylalkohol, Essigsäure, eine Erhöhung der Wirkstoffkonzentration im polymeren Material erreicht wird, auch wenn die Kohäsionsenergiedichte von Polymer und Wirkstoff nicht kompatibel sind.

## Claims

1. A method for the preparation of a prefabricated implantable device made of a polymeric material selected from the group consisting of PDMS, polyurethanes, polycarbonates, polyethylene terephthalates, which contains pharmaceutically active substances, with the exception of antimicrobial agents with quaternary ammonium groups, wherein said prefabricated implantable device is treated with an organic solvent for a given period of time, wherein either said pharmaceutically active substance is simultaneously present, or a pharmaceutically active substance dissolved in a similar or the same organic solvent is treated with the pretreated implant after a given period of time, followed by evaporating the organic solvent or solvents and thereafter optionally sterilizing the treated implants, **characterized in that**

- said organic solvent and said polymeric material have a Flory-Huggins coefficient (polymer interaction factor) of 0.30 < X < 0.605 and/or a similar cohesive energy density as the polymeric material;
- the pharmaceutically active substance to be incorporated has similar cohesive energy densities towards the polymer and the solvent;
- the conditions of evaporation of the organic solvent will determine the release profile of the incorporated active substance in aqueous solution, inasmuch as a rapid removal of the solvent will result in a slow initial release of the active substance from the polymeric matrix, or a slow removal of the solvent will result in a rapid initial release of the active substance from the polymeric matrix, with the further proviso that an implantable device made of:
- PDMS is treated with chloroform and fusidinic acid, erythromycin base, clindamycin stearate, mupirocin, trimetoprim;
- polyurethane is made to swell with DMF, DMA, methyl ethyl ketone, HMPT, DMSO, dioxan (hot), m-cresol, tricresol, n-propanol, dichloroacetic acid or mixtures of acetone/DMF, dichloroethane/diethylene glycol, methanol/carbon tetrachloride, carbon tetrachloride/m-cresol, carbon tetrachloride/cyclohexane, carbon tetrachloride/ethyl acetate, DMF/DMA with various ethers or acetone;
- polycarbonates is made to swell with n-heptane, n-octane, n-hexane, THF, o-dichlorobenzene, benzene, chloroform, acetone, dioxan, DMF, m-cresol, cyclohexanone, pyridine, ethyl acetate, or mixtures, such as chloroform/methanol, methylene chloride/ n-hexane or n-heptane; and
- poly(ethylene terephthalate) is made to swell with cyclohexane/dichloroacetic acid.

2. The method according to claim 1, wherein an increase of the active substance concentration in the polymeric material is achieved with the aid of suitable solubilizers, such as dimethylformamide, ethyl alcohol, acetic acid, even when the cohesive energy densities of polymer and active substance are not compatible.


**Revendications**

1. Procédé de fabrication d'un dispositif implantable préfabriqué en un matériau polymère, choisi dans le groupe composé des PDMS, des polyuréthanes, des polycarbonates, des polyéthylènes téréphtalates, qui contient des principes actifs pharmaceutiques, à l'exception de principes actifs antimicrobiens comportant des groupes ammonium quaternaire, dans lequel le dispositif implantable préfabriqué est, pendant un certain laps de temps, traité à l'aide d'un solvant organique, et dans lequel soit le principe actif pharmaceutique est simultanément présent, soit, au bout d'un certain laps de temps, un principe actif pharmaceutique, dissous dans un solvant organique analogue ou identique, est traité avec l'implant prétraité, puis le ou les solvants organiques sont chassés par évaporation, et les implants traités sont ensuite éventuellement stérilisés, **caractérisé en ce que**

- le solvant organique et le matériau polymère ont un coefficient de Flory-Huggins (Facteur d'Interaction avec le Polymère) $0,30 < X > 0,605$ et/ou une densité d'énergie de cohésion $\delta$ analogue à celle du matériau polymère,
- le principe actif pharmaceutique à incorporer a une densité d'énergie de cohésion analogue à celle du polymère et du solvant,
- les conditions d'évaporation du solvant organique définissent le profil de libération du principe actif incorporé en solution aqueuse, par le fait que, en présence d'une élimination rapide du solvant, on arrive à une libération initiale lente du principe actif à partir de la matrice polymère, ou encore, dans le cas dune élimination lente du solvant, on arrive à une libération initiale rapide du principe actif à partir de la matrice polymère, dans la mesure supplémentaire, où un dispositif implantable en PDMS est traité avec du chloroforme et de l'acide fusidique, de l'érythromycine base, du stéarate de clindamycine, de la mupirocine, du trimétoprim,
- on utilise pour le gonflement de polyuréthannes du DMF, du DMA, de la méthyléthylcétone, du HMPT, du DMSO, du dioxanne (chaud), du m-crésol, du tricrésol, du n-propanol, de l'acide dichloracétique ou des mélanges acétone/DMF, dichloréthane/diéthylèneglycol, méthanol/ tétrachlorure de carbone, tétrachlorure de carbone/m-crésol, tétrachlorure de carbone/cyclohexane, tétrachlorure de carbone/acétate d'éthyle, DMF/DMA avec différents éthers ou de l'acétone,
- on utilise pour le gonflement de polycarbonates du n-heptane, du n-octane, du n-hexane, du THF, de l'odichlorobenzène, du benzène, du chloroforme, de l'acétone, du dioxanne, du DMF, du m-crésol, de la cyclohexanone, de la pyridine, de l'acétate d'éthyle ou des mélanges tels que les mélanges chloroforme/méthanol, chlorure de méthylène/n-hexane ou n-heptane, et
- pour celui du poly(téréphtalate d'éthylène) un mélange cyclohexane/acide dichloracétique.

2. Procédé selon la revendications 1, dans lequel, à l'aide de tiers-solvants appropriés tels que le diméthylformamide, alcool éthylique, l'acide acétique, on arrive à une augmentation de la concentration du principe actif dans le matériau polymère, même quand les densités d'énergie de cohésion du polymère et du principe actif ne sont pas compatibles.

Bestimmung der Löslichkeitsparameter δ von Polymer und Pharmakon

Fig. 1  Bestimmung der Kohäsionsenergiedichte δ des Silikons

Reduzierte Löslichkeit Rifampicin gegen
Kohäsionsenergiedichte

Fig. 2  Bestimmung von δ Rifampicin mittels reduzierter Löslichkeit

Fig. 1 und Fig. 2 sind als visuelle Erklärung der Patentansprüche vorgesehen.